# EUROPEAN PATENT SPECIFICATION

(11) **EP 2 015 714 B1**
(45) Date of publication and mention of the grant of the patent: **05.08.2015**
(21) Application number: 07755776.7
(22) Date of filing: 19.04.2007
(51) Int. Cl.: A61F 2/91, A61F 2/915

(54) **INTRALUMINAL MEDICAL DEVICE HAVING A CURABLE COATING**
INTRALUMINALE MEDIZINISCHE VORRICHTUNG MIT HÄRTBARER BESCHICHTUNG
DISPOSITIF MEDICAL INTRALUMINAL AYANT UN REVETEMENT DURCISSABLE

(30) Priority: 04.05.2006 US 417467
(43) Date of publication of application: 21.01.2009
(73) Proprietor: Boston Scientific Limited, Hamilton HM11 (BM)
(72) Inventor: GREGORICH, Daniel, St. Louis Park, Minnesota 55416 (US); MEYER, Michael, Richfield, Minnesota 55423 (US); TOOLEY, Richard, Crystal, Minnesota 55428 (US); SORENSON, Shawn, Maple Grove, mn 55311 (US)
(74) Representative: Peterreins, Frank
(86) International application number: PCT/US2007/009630
(87) International publication number: WO 2007/130279

(56) References cited:
- WO-A-2004/045462
- WO-A-2005/065550

## Description

### FIELD OF THE INVENTION

The present invention relates to the field of insertable and/or implantable medical devices, in particular stents.

### BACKGROUND OF THE INVENTION

Stents, grafts, stent-grafts, vena cava filters and similar implantable medical devices, collectively referred to hereinafter as stents, are radially expandable endoprostheses which are typically intravascular implants capable of being implanted transluminally and enlarged radially after being introduced percutaneously. Stents may be implanted in a variety of body lumens or vessels such as within the vascular system, urinary tracts, bile ducts, etc. Stents may be used to reinforce body vessels and to prevent restenosis following angioplasty in the vascular system. They may be self-expanding, mechanically expandable or hybrid expandable.

Stents are generally tubular devices for insertion into body lumens. However, it should be noted that stents may be provided in a wide variety of sizes and shapes. Balloon expandable stents require mounting over a balloon, positioning, and inflation of the balloon to expand the stent radially outward. Self-expanding stents expand into place when unconstrained, without requiring assistance from a balloon. A self-expanding stent is biased so as to expand upon release from the delivery catheter. Some stents may be characterized as hybrid stents which have some characteristics of both self-expandable and balloon expandable stents.

Typically, a stent is implanted in a blood vessel or other body lumen at the site of a stenosis or aneurysm by so-called "minimally invasive techniques" in which the stent is compressed radially inwards and is delivered by a catheter to the site where it is required through the patient's skin or by a "cut down" technique in which the blood vessel concerned is exposed by minor surgical means. When the stent is positioned at the correct location, the catheter is withdrawn and the stent is caused or allowed to expand to a predetermined diameter in the vessel.

Despite the wide variety of stents presently available, there remains a desire to provide stents and stent designs which provide a more optimized combination of stent properties including low opening pressure, flexibility in the unexpanded state, and radial rigidity and strength in an expanded state.

WO 2004/045462 A1, which shows all the features of the preamble of claim 1, discloses a prosthesis comprising in its expanded configuration a generally tubular structure formed of one or more fibres. One or more fibres comprise fibre points of intersection. Prior to deployment, fibres comprise a photo curable coating at or near of its intersection in semi-cured form. When delivering an expansion of the endoprothesis by suitable means, the delivery catheter is placed by ultraviolet light delivery catheter. The ultraviolet radiation is delivered via ultraviolet light delivery catheter and the photo curable coating is cured. Ultraviolet light delivery catheter is then removed from the vessel, and endoprosthesis is left in place.

The technical problem of the invention is to provide a stent remaining flexible and easy to deliver and once employed, provides radial rigidity and resistance to compression.

The problem is solved by a stent according to claim 1.

Without limiting the scope of the invention a brief summary of some of the claimed embodiments of the invention is set forth below. Additional details of the summarized embodiments of the invention and/or additional embodiments of the invention may be found in the Detailed Description of the Invention below.

### SUMMARY OF THE INVENTION

The present invention relates to an intraluminal medical device having low opening pressure and radial rigidity or resistance to compression (crush resistance) achieved through the use of strategically placed curable compositions.

The stent remains flexible and easy to deliver and once deployed, the curable composition may be cured so that the stent becomes more radially rigid and resistant to compression.

The invention is also directed a method of providing a stent with radial rigidity including the steps of strategically positioning a curable composition on the stent structure, radially expanding the stent, and curing the composition to form a radially rigid stent structure.

These and other aspects, embodiments and advantages of the present invention will become immediately apparent to those of ordinary skill in the art upon review of the Detailed Description and Claims to follow.

### BRIEF DESCRIPTION OF THE DRAWINGS

FIG. 1A is a flat view of a stent according to the invention in an unexpanded state.
FIG. 1B is a perspective view of a stent similar to that shown in FIG. 1 also in an unexpanded state.
FIG. 2A is a flat view of a stent similar to that shown in FIGS. 1A and 1B in an expanded state.
FIG. 2B is a perspective view of a stent similar to that shown in FIGS. 1A, 1B and 2A in an expanded state.
FIG. 3 is a longitudinal cross-sectional view of a balloon catheter assembly having a stent mounted thereon.
FIGS. 4 and 5 are flat views of alternate stent configurations which may be used in accordance with the invention shown in an unexpanded state.

### DETAILED DESCRIPTION OF THE INVENTION

While this invention may be embodied in many different forms, there are described in detail herein specific preferred embodiments of the invention. This description is an exemplification of the principles of the invention and is not intended to limit the invention to the particular embodiments illustrated.

For the purposes of this disclosure, unless otherwise indicated, identical reference numerals used in different figures refer to the same component.

Expandable stent structures are well known in the art and are available in a wide variety of designs. Some are self-expanding and are delivered to the desired site within a body lumen of a patient and upon removal of a sleeve or other retaining device, self-expand. Others are delivered in a compressed state on an expandable medical balloon and upon expansion of the balloon, the stent expands.

The present invention may be employed with any stent design.

The present invention relates generally to the use of a curable composition strategically positioned on a stent structure which remains in an uncured state until the stent has been delivered to the site of deployment in a body lumen. After expansion, a light can be employed to cure the composition which then hardens and strengthens the curable composition which in turn provides the stent which higher structural strength in an expanded state, thereby increasing its resistance to compression (crush resistance).

As the curable composition is not cured until stent deployment, the stent maintains a low opening pressure and more flexibility during delivery of the stent through the vasculature.

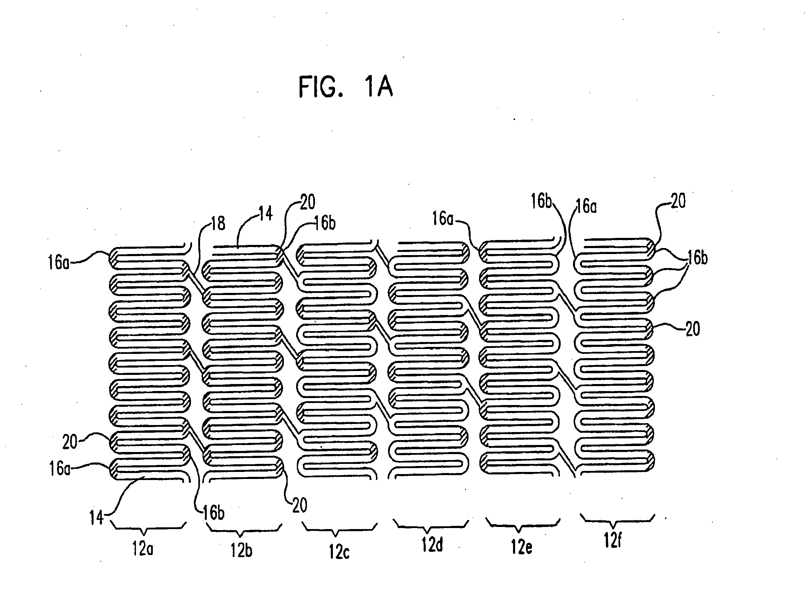

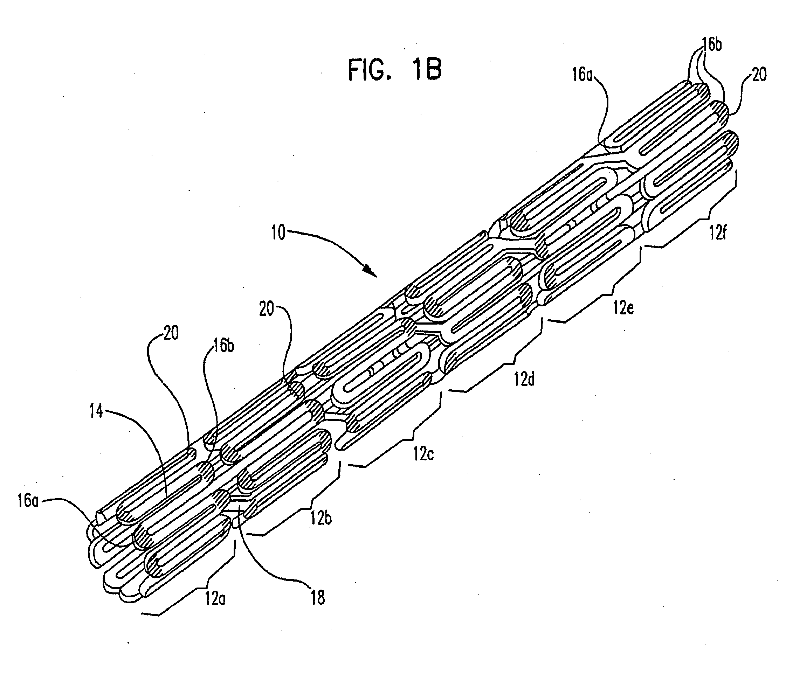

Turning now to the figures, FIG. 1A is a fragmentary flat view of an unexpanded stent 10 according to the invention. Stent 10 is formed of a plurality of serpentine bands 12, each band including straight sections 14 which may be referred to as struts, interconnected by curved end regions 16a, 16b. Each serpentine band 12 is connected by at least one, and suitably a plurality of connectors 18. FIG. 1B is a perspective view of a tubular stent 10 having a similar design to that shown in FIG. 1A.

In this embodiment, a curable resin composition 20, such as a light or ultraviolet (UV) curable resin composition is applied to at least some of the end regions 16a, 16b of the serpentine bands 12 of stent 10. The first two serpentine bands, 12a, 12b, are shown having all of the end portions 16a, 16b with a curable composition disposed thereon. Serpentine bands 12c and 12d, are shown having some end portions 16a, 16b having curable composition 20, disposed thereon. Serpentine bands 12e and 12f are shown wherein end portions 16a have curable composition 20 disposed thereon and wherein end portions 16b have curable composition 20 disposed thereon, respectively.

Curable resin composition 20 may be applied using any suitable method known in the art including, but not limited to, extrusion and spray applications, for example.

The curable resin composition 20 remains uncured while the stent 10 is in an unexpanded configuration, such as during deployment of the stent 10 through a patient's body lumen.

Stent 10 may be delivered to the deployment site using a catheter delivery system. If the stent 10 is self-expanding, sleeves or other retaining devices may restrain the stent at the distal end of the catheter assembly. If the stent is a balloon expandable stent, then it may be crimped onto the expandable balloon member located at the distal end of an elongate balloon catheter assembly as is known in the art.

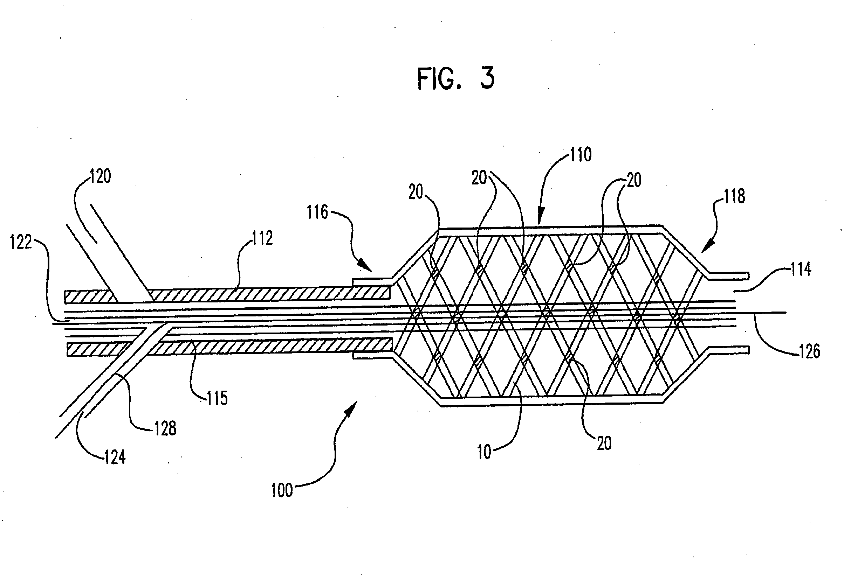

FIG. 3 is a longitudinal cross-sectional view of a balloon catheter assembly 100 having an expandable balloon 110 located at the distal end of the balloon catheter assembly and wherein the balloon is mounted to an elongated outer shaft 112 at the proximal end 116 and mounted to an elongated inner shaft 114 at the distal end 118 of the balloon 110. The catheter assembly if further equipped with an inflation port 120, a guide wire port 122 and a port 124 for a light. Guide wire 126 is shown extending through the lumen of the inner shaft 114. Light 128 is also shown extending through the lumen of the inner shaft 114. One example of a suitable light 128 is a fiber optic cable. The fiber optic cable acts as a light guide and the light source for the fiber optic cable may be any suitable light source, for example, light emitting diode (LED) or a laser. As used herein, the light shall refer to that portion which is advanced through the lumen and is employed for curing the curable composition on the stent at the lesion site while the light source remains outside of the patient's body.

A separate light lumen may be incorporated into the catheter shaft assembly, or a single lumen can be employed for both inflation and for the light. In the latter case, the shaft which forms the lumen, as well as the inflation fluid, are desirably formed from a clear (transparent) material such as a clear polymeric material which is transparent to the type of radiation being employed for curing the curable composition.

Some methods of delivering light inside the body of a patient are disclosed in U.S. Patent Nos. 5997570, 5591199, 6485512 and 6869442.

As shown in FIG. 3, the light 124 is shown being advanced through the same lumen as the guide wire. In this instance, a clear inner shaft would be advantageous. However, the catheter assembly may be modified so as to include a separate lumen for light 124, or the light 124 may be advanced through the inflation lumen 115 as well.

Stent 10 is shown mounted on expandable balloon member 110. Stent 10 is shown having a generic configuration and is further shown having a curable resin composition 20 located strategically on the stent 10.

In some embodiments of the invention composition 20 may be located at other locations on the stent 10 in addition and/or alternatively to the end portions previously described. In at least one embodiment for example, composition 20 is positioned on selected end portions described but also on one or more portions of a strut or struts extending therebetween. Moreover, location(s) of the composition 20 on the stent 10 may be on any surface of the stent or combination of surfaces. In at least one embodiment for example, composition 20 is positioned on the proximal surface, distal surface, inner surface, outer surface or any combination of surfaces of the stent in the region of intersection between two or more stent members such as struts and/or connectors.

Stent 10 may be delivered to the deployment site within a patient's body lumen with stent 10 in an unexpended state as shown in FIGS. IA and IB5 and with curable resin composition 20 in an uncured state. The uncured resin composition allows the stent to maintain a low opening pressure and flexibility.

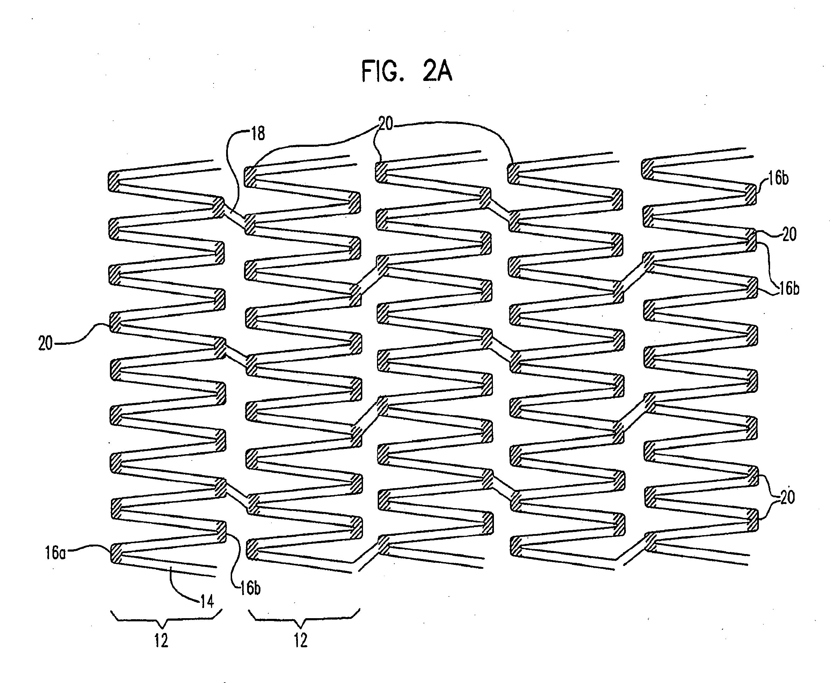

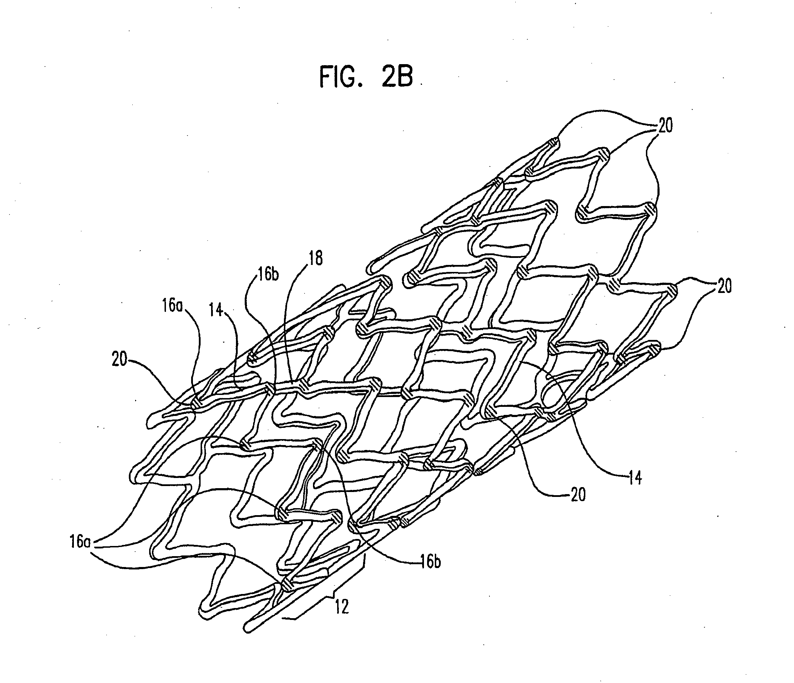

Once at the site of deployment, stent 10 may be expanded. FIG. 2A is partial flat view of a stent having a similar design to those shown in FIGS. IA and IB shown in an expanded state and FIG. 2B is a perspective view of a tubular stent of a similar design shown in an expanded state.

In this embodiment, the curable resin composition 20 is shown coated on substantially all of the end portions.

While the curable resin composition 20 is shown coated on the outside of the end portions 16a, 16b of the serpentine band 12, the curable composition 20 may be coated on the outside, inside, or both of each end portion 16a, 16b.

The strategically placed curable coating composition of the invention may be employed on any stent design where it is desired to increase crush resistance, while maintaining low opening pressure for stent expansion and maintaining flexibility for delivery of the stent to the deployment site within a body lumen. Some examples of stent designs arc found in commonly assigned U.S. Patent No. 6776793. Other stent designs are found in commonly assigned copending Patent Application Publication Nos. 20040073290 and 20050182480. Such examples are intended for illustrative purposes only and are not intended to limit the scope of the present invention.

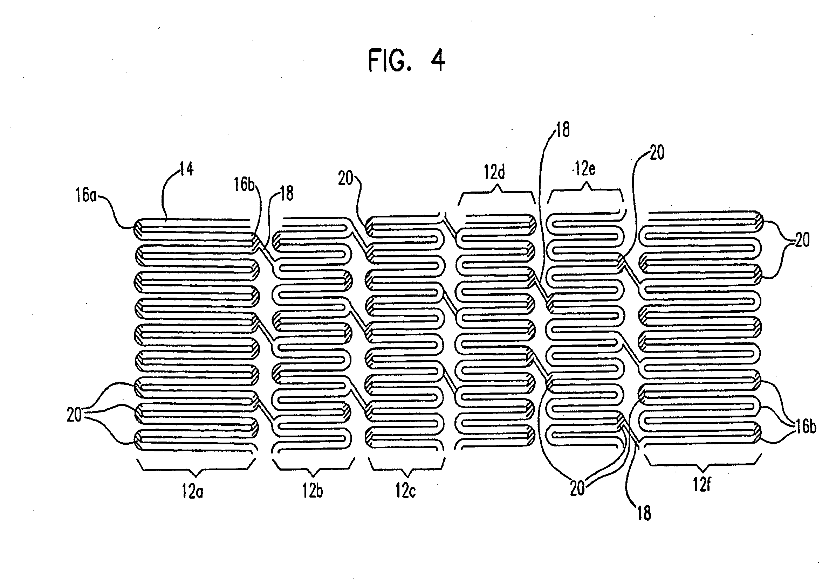

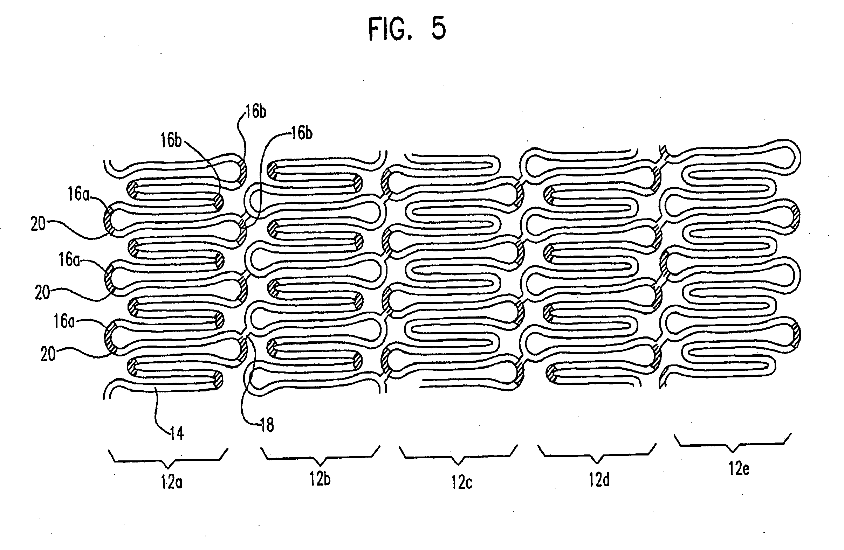

FIGS. 4 and 5 are partial flat views of alternative stent designs. Furthermore, each serpentine band in FIG. 4, illustrates alternative placement of the curable composition 20 on the end portions. For example, serpentine band 12a, shows every end portion 16a, 16b having curable composition 20 thereon. Serpentine band 12b illustrates placement of the curable composition 20 on alternate end portions 16a, 16b. Serpentine band 12c illustrates having curable composition 20 on end portions 16a on one side of serpentine band 12c and serpentine band 12d illustrates having curable composition 20 on end portions 16b only. Serpentine band 12e shows curable composition 20 placed on those end portions which arc further attached by a connector 18 and serpentine band 12f shows a more random placement of curable composition 20.

Each serpentine band in FIG. 5, 12a-12f, also show a variety of different ways of coating end portions with some end portions being coated in 12b-12e and all of the end portions shown coated in 12a. This is intended for illustrative purposes only, and not as a limitation on the scope of the present invention.

The above examples are intended for illustrate purposes only and not as a limitation on the scope of the present invention.

Any suitable curable resin composition may be employed herein. Examples of materials which cure upon exposure to radiation include, but are not limited to, urethanes, polyurethane oligomer mixtures, acrylate monomers, aliphatic urethane acrylate oligomers, acrylamides, UV curable epoxies, etc.

Specific examples of suitable UV curable materials include the Dymax® polyurethane oligomer mixture optical and fiber optic resins available from Dymax in Torrington, CT. These resins can cure within one to ten seconds once exposed to ultraviolet light and provide a good tensile strength of 20,7 MPa (3000 psi) or more. Dymax also offers the equipment which can be employed to provide the UV radiation for curing.

Acrylate/methacrylate monomer/oligomer and mixtures of monomers and oligomers are available from Sartomer in Exton, PA. Aliphatic and aromatic urethane (meth)acrylate oligomers, amine modified acrylate oligomers, epoxy (meth)acrylates, silicone acrylate oligomers, etc. are also available from Sartomer.

Acrylamides are available from Loctite Corp. in Newington, CT.

The appropriate type of radiation and length of exposure required will vary depending on the type of material selected.

While it is preferable to employ a UV curable material, other types of radiation such as visible, infrared and thermal radiation can also be employed. One particular example of a light source is either a UV light source or an excimer laser which can provide radiation having a wavelength from about 240 to about 400 nm, such as a KrF laser which operates at about 248 nm. Other lasers include a neodymium doped YAG laser which operates at about 266 nm. Argon ion lasers may also be employed.

Chemically curable materials such as silicone-based compounds which undergo room temperature vulcanization (RTV) may also be employed. Furthermore, moisture curable materials such as moisture curable silicone based compounds such as siloxanes, moisture curing silanes, moisture curable urethanes, polyurethane oligomers and mixtures thereof, moisture curing acrylic urethanes, etc. can also be employed herein.

Some materials also cure slowly over time such as epoxies, and may also be employed herein.

Suitably, the composition is selected so as to have adhesive properties such that the composition forms a sufficient bond to the stent. Adhesion can be improved by first treating the surface of the stent with methods known in the art.

Some examples of suitable materials are disclosed in US 5591199 (see also EP 0 836448).

A biocompatible coating may be further used to encapsulate the curable resin composition. Examples of suitable biocompatible materials include, but are not limited to, silicones, polyacrylamides, polyolefins such as polyethylene and polypropylene and copolymers of ethylene and propylene, polystyrene and copolymers of styrene, polyurethanes, etc.

The above lists are intended for illustrative purposes only, and are not intended to limit the scope of the present invention. Any curable compound which exhibits increased strength and rigidity may be employed herein. Lubricious coatings may also be employed.

Therapeutic agents may also be incorporated therein, such as in the biocompatible coating or in the curable coating.

The stents may be made as is known in the art. Any suitable stent material may be used in the manufacture of the inventive stents such as metals and metal alloys including, but not limited to, stainless steel, cobalt chrome alloys such as elgiloy, tantalum or other plastically deformable metals, shape-memory metals such as nickel- titanium alloys generically known as "nitinol", platinum/tungsten alloys and titanium alloys. Polymers and ceramics may also be employed.

The invention also contemplates the use of more than one material in the inventive stents. For example, the first undulating bands and the second undulating bands may be made of different materials. Optionally, the connectors may be made of a different material than the first and/or second undulating bands.

The inventive stents may be provided in mechanically expandable form, in self-expanding form or as a hybrid of the two. Mechanically expandable stents, in accordance with the invention, may be expanded using any suitable mechanical device including a balloon.

The inventive stents may be manufactured using known stent manufacturing techniques. Suitable methods for manufacturing the inventive stents include laser cutting, chemical etching or stamping of a tube. The inventive stents may also be manufactured by laser cutting, chemically etching, stamping a flat sheet, rolling the sheet and welding the sheet, by electrode discharge machining, or by molding the stent with the desired design.

Further, the inventive stents may include coatings for improved visibility, for example, suitable radiopaque coatings. Examples of radiopaque materials include, but are not limited to, gold or other noble metals or sputtered with tantalum or other metals. The stents may also be made directly from a radiopaque material to obviate the need for a radiopaque coating or may be made of a material having a radiopaque inner core. Other radiopaque metals which may be used include platinum, platinum-tungsten, palladium, platinum-indium, rhodium, tantalum, or alloys or composites of these metals.

The inventive stents may also be provided with various bio-compatible coatings to enhance various properties of the stent. For example, the inventive stents may be provided with lubricious coatings. The inventive stents may also be provided with drug-containing coatings which release drugs over time.

The inventive stents may also be provided with a sugar or more generally a carbohydrate and/or a gelatin to maintain the stent on a balloon during delivery of the stent to a desired bodily location. Other suitable compounds for treating the stent include biodegradable polymers and polymers which are dissolvable in bodily fluids. Portions of the interior and/or exterior of the stent may be coated or impregnated with the compound. Mechanical retention devices may also be used to maintain the stent on the balloon during delivery.

The inventive stents may also be used as the framework for a graft. Suitable coverings include nylon, collagen, PTFE and expanded PTFE, polyethylene terephthalate and KEVLAR, or any of the materials disclosed in U.S. Pat. Nos. 5,824,046 and 5,755,770. More generally, any known graft material may be used including synthetic polymers such as polyethylene, polypropylene, polyurethane, polyglycolic acid, polyesters, polyamides, their mixtures, blends, copolymers, mixtures, blends and copolymers.

The inventive stents may find use in coronary arteries, renal arteries, peripheral arteries including illiac arteries, arteries of the neck and cerebral arteries. The stents of the present invention, however, are not limited to use in the vascular system and may also be advantageously employed in other body structures, including but not limited to arteries, veins, biliary ducts, urethras, fallopian tubes, bronchial tubes, the trachea, the esophagus and the prostate.

While in the embodiments shown above there are at least three interconnecting elements joining adjacent first and second bands although fewer or additional interconnecting elements are also contemplated.

It is understood that the peaks and troughs of the present invention need not be rounded, as shown in the Figures. The peaks and troughs may be bulbous, triangular, square, pointed, or otherwise formed of interconnected straight sections.

As already indicated, this invention is applicable to self-expanding configurations, mechanically expandable configurations and to a wide variety of materials, including both metal and plastic and any other material capable of functioning as an expandable stent. For example, the stent may be of metal wire or ribbon such as tantalum, stainless steel or the like. It may be thin-walled. It may be of shape memory alloy such as Nitinol or the like, etc. The interconnecting elements may be formed integrally with the band-like elements (or segments) or may be bonded thereto via such methods as adhesive bonding, welding or any other known method of bonding.

## Claims

1. A stent (10) comprising a plurality of serpentine bands (12), each band formed from a plurality of straight sections (14) connected by curved end portions (16 a, 16 b), at least some of the end portions having a curable composition. (20) disposed thereon, **characterised in that** the curable composition (20) is positioned on the curved end portions only, wherein said curable composition is strategically positioned on all or on alternate end portions (16a, 16b) or on end portions (16a, 16b), which are further attached by a connector (18), or wherein the curable composition (20) is randomly placed on the end portions (16a, 16b), or wherein one side of a serpentine band (20) of the plurality of serpentine bands (12) is provided with the curable composition (20) on end portions (16a) only, and another serpentine band (20) of the plurality of serpentine bands (12) is provided with the curable composition (20) on end portions (16b) only.

2. The stent of claim 1 wherein all of the end portions (16) have a curable composition (20) disposed thereon.

3. The stent of claim 1 wherein said curable composition (20) is cured upon exposure to ultraviolet radiation.

4. The stent of claim 1 wherein said curable composition (20) is cured with a laser having a wavelength between about 240 run and 400 nm.

5. The stent of claim 1 wherein said curable composition (20) comprises at least one member selected from the group consisting of urethanes, polyurethane oligomers, (meth)acrylates, aliphatic urethane acrylate oligomers, aromatic urethane acrylate oligomers, acrylamides, epoxies and mixtures thereof.

6. The stent of claim 1 wherein said curable resin composition comprises at least one urethane monomer, urethane oligomer or mixture there.

7. The stent of claim 1 further comprising a biocompatible coating.

8. The stent of claim 1 further comprising a therapeutic agent in said curable composition of in said biocompatible coating.

9. The stent of claim 1 in combination with a catheter assembly (100), the catheter assembly (100) having a distal end and a proximal end, the stent mounted on the distal end, the catheter assembly further comprising a lumen for advancement of a light for curing said curable resin composition.

10. The stent of claim 9 wherein the lumen for the advancement of a light is the same as the inflation lumen.

11. The stent of claim 1 wherein said stent (10) is balloon inflatable.

## Patentansprüche

1. Stent (10) umfassend eine Vielzahl an gewundenen Bändern (12), wobei jedes Band aus einer Vielzahl an geraden Abschnitten (14) geformt ist, die durch gebogene Endstücke (16a, 16b) miteinander verbunden sind, wobei zumindest einige der Endstücke eine härtbare Zusammensetzung (20) haben, die darauf angeordnet ist, **dadurch gekennzeichnet, dass** die härtbare Zusammensetzung (20) nur auf den gebogenen Endstücken positioniert ist, wobei die härtbare Zusammensetzung strategisch auf allen oder auf alternierenden Endstücken (16a, 16b) oder auf Endstücken (16a, 16b) positioniert ist, die außerdem über ein Verbindungsstück (18) verbunden sind, oder wobei die härtbare Zusammensetzung (20) willkürlich auf den Endstücken (16a, 16b) platziert ist, oder wobei nur eine Seite eines gewundenen Bands (20) der Vielzahl an gewundenen Bändern (12) mit der härtbaren Zusammensetzung (20) auf den Endstücken (16a) versehen ist, und ein anderes gewundenes Band (20) der Vielzahl an gewundenen Bändern (12) mit der härtbaren Zusammensetzung (20) nur auf Endstücken (16b) versehen ist.

2. Stent nach Anspruch 1, wobei alle der Endstücke (16) eine härtbare Zusammensetzung (20) haben, die darauf angeordnet ist.

3. Stent nach Anspruch 1, wobei die härtbare Zusammensetzung (20) durch die Einwirkung ultravioletter Strahlung gehärtet wird.

4. Stent nach Anspruch 1, wobei die härtbare Zusammensetzung (20) mit einem Laser gehärtet wird, der eine Wellenlänge zwischen ungefähr 240 nm und 400 nm hat.

5. Stent nach Anspruch 1 wobei die härtbare Zusammensetzung (20) mindestens einen Teil umfasst, der aus der Gruppe ausgewählt ist, die besteht aus: Urethanen, polyurethanen Oligomeren, (Meth)acrylaten, aliphatischen Urethan-Acrylat-Oligomeren, aromatischen Urethan-Acrylat-Oligomeren, Acrylamiden, Epoxiden und Gemischen davon.

6. Stent nach Anspruch 1, wobei die härtbare Harzzusammensetzung mindestens ein Urethan-Monomer, Urethan-Oligomer oder ein Gemisch davon umfasst.

7. Stent nach Anspruch 1, außerdem umfassend eine biokompatible Beschichtung.

8. Stent nach Anspruch 1, außerdem umfassend einen therapeutischen Wirkstoff in der härtbaren Zusammensetzung oder in der biokompatiblen Beschichtung.

9. Stent nach Anspruch 1 in Kombination mit einer Kathetereinheit (100), wobei die Kathetereinheit (100) ein distales Ende und ein proximales Ende hat, wobei der Stent an einem distalen Ende angebracht ist, wobei die Kathetereinheit außerdem ein Lumen umfasst, für die Weiterleitung von Licht zum Härten der härtbaren Harzzusammensetzung.

10. Stent nach Anspruch 9, wobei das Lumen für die Weiterleitung des Lichts das gleiche ist wie das Inflationslumen.

11. Stent nach Anspruch 1, wobei der Stent (10) Ballon inflatierbar ist.

## Revendications

1. Stent (10) comprenant une pluralité de bandes en serpentin (12), chaque bande étant constituée d'une pluralité de sections droites (14) connectées par des parties d'extrémité courbes (16a, 16b), au moins plusieurs des parties d'extrémité comportant une composition durcissable (20) disposée sur celles-ci, **caractérisé en ce que** la composition durcissable (20) est positionnée sur les parties d'extrémité courbes uniquement, dans lequel ladite composition durcissable est positionnée de façon stratégique sur la totalité des parties d'extrémités (16a, 16b) ou sur des parties d'extrémité alternées (16a, 16b) ou sur des parties d'extrémité (16a, 16b), qui sont en outre attachées par un connecteur (18), ou dans lequel la composition durcissable (20) est placée de façon aléatoire sur les parties d'extrémité (16a, 16b), ou dans lequel un côté d'une bande en serpentin (20) de la pluralité de bandes en serpentin (12) comporte la composition durcissable (20) sur des parties d'extrémité (16a) uniquement, et une autre bande en serpentin (20) de la pluralité de bandes en serpentin (12) comporte la composition durcissable (20) sur des parties d'extrémité (16b) uniquement.

2. Stent selon la revendication 1, dans lequel toutes les parties d'extrémité (16) comportent une composition durcissable (20) qui est disposée sur celles-ci.

3. Stent selon la revendication 1, dans lequel ladite composition durcissable (20) est durcie lors d'une exposition à un rayonnement ultraviolet.

4. Stent selon la revendication 1, dans lequel ladite composition durcissable (20) est durcie par un laser présentant une longueur d'onde comprise entre environ 240 nm et 400 nm.

5. Stent selon la revendication 1, dans lequel ladite composition durcissable (20) comprend au moins un élément qui est sélectionné dans le groupe comprenant des uréthanes, des oligomères de polyuréthane, des (méth)acrylates, des oligomères d'acrylate d'uréthane aliphatique, des oligomères d'acrylate d'uréthane aromatique, des acrylamides, des époxy ainsi que des mélanges de ceux-ci.

6. Stent selon la revendication 1, dans lequel ladite composition de résine durcissable comprend au moins un monomère d'uréthane, un oligomère d'uréthane ou un mélange de ceux-ci.

7. Stent selon la revendication 1, comprenant en outre un revêtement biocompatible.

8. Stent selon la revendication 1, comprenant en outre un agent thérapeutique dans ladite composition durcissable dans ledit revêtement biocompatible.

9. Stent selon la revendication 1 en combinaison avec un ensemble de cathéter (100), l'ensemble de cathéter (100) présentant une extrémité distale et une extrémité proximale, le stent étant monté sur l'extrémité distale, l'ensemble de cathéter comprenant en outre une lumière pour l'avancement d'une lumière pour faire durcir ladite composition de résine durcissable.

10. Stent selon la revendication 9, dans lequel la lumière pour l'avancement d'une lumière est la même que la lumière de gonflage.

11. Stent selon la revendication 1, dans lequel ledit stent (10) est gonflable par ballonnet.
